# EUROPEAN PATENT APPLICATION

(11) **EP 4 257 071 A1**
(43) Date of publication of application: **11.10.2023**
(21) Application number: 20964059.8
(22) Date of filing: 05.12.2020
(51) Int. Cl.: A61B 34/30, A61B 17/29, B25J 17/00, B25J 19/00

(54) **QUICK INSERTION-AND-REMOVAL DEVICE, ACTUATING MECHANISM AND SURGICAL ROBOT**

(71) Applicant: Noahtron Intelligence Medtech (Hangzhou) Co., Ltd., Hangzhou, Zhejiang 310051 (CN)
(72) Inventor: PAN, Lufeng, Hangzhou, Zhejiang 310051 (CN); HUANG, Shandeng, Hangzhou, Zhejiang 310051 (CN); LIU, Jianfei, Hangzhou, Zhejiang 310051 (CN); YAN, Yongli, Hangzhou, Zhejiang 310051 (CN)
(74) Representative: Petraz, Gilberto Luigi
(86) International application number: PCT/CN2020/134110
(87) International publication number: WO 2022/116214

(57) **Abstract**

A quick insertion-and-removal device comprises: a base connected to a surgical instrument; an outer sheath limiting assembly comprising a limiting element capable of moving relative to the base, wherein the limiting element is configured to lock or unlock an outer sheath; a push-rod limiting assembly comprising a sliding sleeve capable of moving relative to the base to lock or unlock a push rod; and a mounting-dismounting control assembly comprising a joint unlocking member, wherein the joint unlocking member is connected to the sliding sleeve and can drive the sliding sleeve to move, relative to the base, to a position where the push rod is unlocked, and in this position, the joint unlocking member is engaged with the limiting element and can unlock the outer sheath.

## Description

### TECHNICAL FIELD

The disclosure relates to the technical field of medical devices, in particular to a quick insertion-and-removal device, an executing mechanism and a surgical robot.

### DESCRIPTION OF THE PRIOR ART

In minimally invasive surgery, a tiny wound is opened in the patient's body, and part of an executing mechanism of a surgical robot passes through the tiny wound and enters the lesion site, with the telecentric fixed point of the executing mechanism coinciding with the wound; in addition to the executing mechanism's own operations, the operator controls the arm of the robot to drive the executing mechanism to swing about the telecentric fixed point as the hinge point within a certain angle range to complete the minimally invasive surgery. Minimally invasive surgery has gradually gained favor from medical staffs and patients in recent years due to its tiny wound and less bleeding.

The executing mechanism generally includes: a surgical instrument used to extend into the lesion, and a driving assembly used to drive the surgical instrument to rotate, open and close, among others. Driven by the driving assembly, the operating end of the surgical instrument extending into the human body completes the preset surgical operations. In order to complete different surgical operations, the surgical instrument needs to be replaced accordingly. However, in the existing executing mechanisms, the mounting and dismounting of surgical instrument is not convenient enough, and it is difficult to meet the requirement of quick mounting and dismounting of surgical instrument.

### SUMMARY OF THE DISCLOSURE

In view of this, it is necessary to provide a quick insertion-and-removal device, an executing mechanism and a surgical robot, wherein the quick insertion-and-removal device can realize quick mounting and dismounting of surgical instrument.

Embodiments of the present disclosure firstly provide a quick insertion-and-removal device for a surgical instrument, including:
a base for slidably connecting the surgical instrument;
an outer sheath limiting assembly provided on the base and including a limiting element, the limiting element is movable relative to the base in a direction perpendicular to a sliding direction of the surgical instrument and used for locking or unlocking an outer sheath of the surgical instrument;
a push rod limiting assembly provided on the base and including a sliding sleeve which is movable relative to the base to lock or unlock a push rod of the surgical instrument; and
a mounting-dismounting control assembly including a joint unlocking member, the joint unlocking member is movable relative to the base and connected to the sliding sleeve and capable of driving the sliding sleeve to move relative to the base to a position of unlocking the push rod, where the joint unlocking member engages with the limiting element and is capable of unlocking the outer sheath.

When dismounting and replacing a surgical instrument, both the outer sheath and the push rod need to be unlocked and then slide relative to the base to be removed. In the above quick insertion-and-removal device, the joint unlocking member of the mounting-dismounting control assembly drives the sliding sleeve of the push rod limiting assembly to slide, thereby unlocking the push rod of the surgical instrument. Further, at the position where the push rod is unlocked, the joint unlocking member can unlock the outer sheath simultaneously or subsequently. In other words, through the movement of the joint unlocking member relative to the base, both the outer sheath and the push rod can be unlocked, no need to separately control two unlocking mechanisms. The mounting and dismounting of the surgical instrument is relatively simple, facilitating the rapid replacement of the surgical instrument.

In a feasible implementation, the base has a slide hole extending perpendicular to a sliding direction of the outer sheath, the limiting element is slidably connected to the slide hole and opened with an outer sheath limiting hole and is capable of locking or unlocking the outer sheath of the surgical instrument through the outer sheath limiting hole.

Further, when the joint unlocking member moves to a position of being engaged with the limiting element, the joint unlocking member is capable of driving the limiting element to slide in the slide hole to unlock the outer sheath.

The limiting element is slidably provided on the base, so that when the joint unlocking member is engaged with the limiting element, the user can drive the limiting element to slide in the slide hole of the base through the joint unlocking member, and then the outer sheath can be unlocked, which can avoid an unreliable installation of the outer sheath resulted from a touch of the joint unlocking member by mistake.

In a feasible implementation, the mounting-dismounting control assembly further includes a shift block connector, one side of which is fixedly connected to the joint unlocking member, and the other side extends along a motion direction of the joint unlocking member to form as a slidable portion.

In this way, the slidable portion cooperates with the base to limit the tilting or rolling of the shift block connector, so that the shift block connector can drive the joint unlocking member to move more stably relative to the base, thereby unlocking the surgical instrument more stably and reliably.

In a feasible implementation, a protrusion is provided on one side of the base; an accommodating hole is defined in the slidable portion for accommodating the protrusion; and the shift block connector is slidable relative to the base on a surface where a top face of the protrusion is located, and when the shift block connector slides with the joint unlocking member to the position where the joint unlocking member is engaged with the limiting element, the accommodating hole accommodates the protrusion as the shift block connector approaches the base.

In this way, before the protrusion is aligned with the accommodating hole, the top surface of the protrusion supports the sliding shift block connector, where the joint unlocking member fixedly connected with the shift block connector has not yet engaged with the sliding sleeve or has not exactly engaged with the sliding sleeve. When the protrusion is aligned with the accommodating hole, the accommodating hole can be located around the outside of the protrusion, where the shift block connector approaches the side of the base as the accommodating hole receives the protrusion, so that the joint unlocking member can drive the limiting element to slide in the slide hole of the base, thereby unlocking the outer sheath.

In a feasible implementation, a plurality of protrusions are provided at intervals along a direction in which the shift block connector slides relative to the base.

In a feasible implementation, the accommodating holes correspond to the protrusions in number and location.

The top surface of the protrusion is used to support the sliding shift block connector, and a plurality of protrusions can make the sliding process of the shift block connector more stable.

In a feasible implementation, a slideway extending along the motion direction of the joint unlocking member is opened in the base, and sides of the slideway are capable of guiding the sliding slidable portion.

At least one side of the slideway can guide the sliding slidable portion of the shift block connector, thereby limiting the deflection of the shift block connector relative to the base and making the sliding process more stable.

In a feasible implementation, an inclined surface is further provided on a bottom surface of the slideway, and the shift block connector is slidable along the inclined surface to approach a side of the base.

As the accommodating hole is approaching to receive the protrusion, the shift block connector gradually slides along the inclined surface to abut the side of the base until the accommodating hole exactly receives the protrusion. The inclined surface allows the limiting element to be gradually driven to slide, which makes the unlocking process more smooth and stable.

In a feasible implementation, the outer sheath limiting assembly further includes a detection element which is arranged within a sliding path of the limiting element and used to detect a stop position of the limiting element in the slide hole.

When the limiting element is driven to slide to the position of unlocking the outer sheath, the user can pull the entire surgical instrument out of the base, and the detection element is used to detect the stop position of the limiting element, so that the user can accurately judge the unlocking status of the surgical instrument through the optical signal, beep or the like fed back from the detection element, so as to determine the timing of pulling out the surgical instrument more accurately, avoiding damaging the associated structures of the surgical instrument or the base.

In a feasible implementation, the outer sheath limiting assembly further includes a first spring which is provided so that the limiting element can be retained in position of locking the outer sheath.

The spring force from the first spring retains the limiting element at the position of locking the outer sheath. Therefore, when no external force is applied, the outer sheath can be more reliably retained in the locked state, preventing usage problems caused by the user forgetting to reset the limiting element after mounting the surgical instrument.

In a feasible implementation, the outer sheath limiting assembly further includes a blocking piece, and the first spring is compressed between the limiting element and the blocking piece. The first spring is compressed between the blocking piece and the limiting element, which can simplify the installation of the first spring.

In a feasible implementation, the limiting element is provided with a first linked engagement portion, the joint unlocking member is provided with a second linked engagement portion, and the joint unlocking member and the limiting element are engageble with each other through cooperation between the first linked engagement portion and the second linked engagement portion.

In a feasible implementation, one of the first linked engagement portion and the second linked engagement portion is configured as an insertion hole, and the other is configured as an insertion portion receivable within the insertion hole.

As the insertion portion of the joint unlocking member is inserted into the insertion hole of the limiting element, the joint unlocking member and the limiting element can be reliably engaged with each other.

In a feasible implementation, the joint unlocking member further includes a sleeving portion for surrounding or being inserted in the sliding sleeve, and the sliding sleeve includes a blocking portion engageble with the sleeving portion so that the sliding sleeve is slidable along with the joint unlocking member.

The engagement between the sleeving portion and the blocking portion can make the sliding sleeve move reliably with the movement of the joint unlocking member.

In a feasible implementation, the push rod limiting assembly further includes a second spring and a spring force from the second spring makes the sliding sleeve keep the tendency of locking the push rod. The spring force from the second spring retains the sliding sleeve at the position of locking the push rod, which can simplify the mounting of the surgical instrument.

In a feasible implementation, the push rod limiting assembly further includes a push rod locking sleeve arranged in the sliding sleeve and a push rod locking member, and wherein the push rod is provided in the push rod locking sleeve; the push rod locking member can be constrained by the sliding sleeve between the push rod locking sleeve and the push rod, so that the push rod is locked relative to the push rod locking sleeve; and when the joint unlocking member moves to the position of being engaged with the limiting element, the sliding sleeve releases the push rod locking member to unlock the push rod.

In a feasible implementation, along a motion direction of the joint unlocking member, the push rod locking members are arranged in pairs and symmetrically distributed on two sides of the push rod, respectively.

Each pair of push rod locking members limits the push rod on two sides of the push rod in the axial direction, which can balance the forces on the push rod from the push rod locking members, improving the mounting and moving precision.

In a feasible implementation, the push rod locking member is configured as a spherical member, and the sliding sleeve can press the spherical member into a locking groove of the push rod to lock the push rod.

Further, a locking hole for accommodating the spherical member is defined in the push rod locking sleeve, and the spherical member is movably arranged within the locking hole.

The push rod locking member configured as the spherical member can move freely in the locking hole, so as to flexibly switch between two positions of locking and unlocking the push rod.

In a feasible implementation, along a sliding direction of the sliding sleeve relative to the push rod locking sleeve, the sliding sleeve has a locking section and an unlocking section, and wherein when the sliding sleeve slides to a position where the locking section corresponds to the push rod locking sleeve, the push rod locking member locks the push rod; and when the sliding sleeve slides to a position where the unlocking section corresponds to the push rod locking sleeve, the push rod locking member is released to unlock the push rod.

In a feasible implementation, one of the push rod locking sleeve and the sliding sleeve is provided with a guiding rib, and the other is provided with a guiding groove in a sliding fit with the guiding rib. The sliding fit between the guiding rib and the guiding groove allows the sliding sleeve to slide relative to the push rod locking sleeve more stably and accurately.

In a feasible implementation, two push rods are provided, each of which corresponds to one said push rod locking sleeve.

Although a single push rod cooperating with an appropriate linkage mechanism can actuate the surgical tool, two push rods driving respective swing members of the surgical tool can increase the freedom of motion of the surgical tool.

The second aspect according to the embodiments of the present disclosure further provides an executing mechanism of a surgical robot, including the quick insertion-and-removal device according to any of the above-mentioned embodiments, and a surgical instrument. The surgical instrument includes an outer sheath and two push rods slidably inserted in the outer sheath, with each of the push rods being connected to a swing member.

In a feasible implementation, the base has a slide hole extending perpendicular to a sliding direction of the outer sheath; the outer sheath limiting assembly includes a limiting element which is slidably connected to the slide hole, and the limiting element is provided with an outer sheath limiting hole; and an outer wall of the outer sheath is opened with an annular limiting groove for limiting part of a hole wall of the outer sheath limiting hole. The annular limiting groove cooperates with part of the hole wall of the outer sheath limiting hole, so that in the locked position, the limiting element and the outer sheath are interfered with each other when axially sliding relative to each other, and thus the outer sheath is locked more reliably.

In a feasible implementation, the executing mechanism further includes two groups of push rod driving assemblies each corresponding to each of the push rods. The push rod driving assembly includes a linear driving device arranged on the base; and a loading seat connected to an output end of motion of the linear driving device for driving the push rod to axially slide.

In a feasible implementation, the linear driving device is configured as a linear motor; and the push rod driving assembly further includes a guiding member which is arranged on the base and used to limit rotation of the loading seat along with the linear motor.

The linear motor drives the loading seat to move linearly, and the loading seat is used to drive the push rod to move linearly along therewith, so that the push rod can telescopically slide with high precision. The linear motor generally includes a common motor for outputting rotary motion and a motion conversion mechanism that converts rotational motion into translational motion, such as a screw-nut assembly. Therefore, the guiding member can limit the rotation of the loading seat.

In a feasible implementation, the push rod limiting assembly includes two push rod locking sleeves arranged within the sliding sleeve, and in the locked state, the push rod can move with the push rod locking sleeve which is connected to the loading seat.

The push rod is connected to the loading seat through the push rod locking sleeve, so that when replacing the surgical instrument, it is only necessary to unlock the push rod relative to the push rod locking sleeve, which is more convenient for mounting and dismounting.

In a feasible implementation, the push rod driving assembly further includes an assembly transition plate, and the loading seat is connectable with the push rod locking sleeve through the assembly transition plate. The assembly transition plate facilitates the assembly of the linear motor and the push rod locking sleeve.

In a feasible implementation, the executing mechanism further includes two groups of push rod driving assemblies each corresponding to each of the push rods. The push rod driving assembly includes a linear driving device directly arranged on the base; and a push rod locking sleeve connected to an output end of motion of the linear driving device for driving the push rod to axially slide. The linear driving device which is directly arranged on the base has a higher installation accuracy than that is indirectly arranged on the base through other parts, so that the push rod locking sleeve moving along with the linear driving device also has a higher motion precision.

The third aspect according to the embodiments of the present disclosure further provides a surgical robot, including the above-mentioned executing mechanism, and a telecentric manipulating mechanism. The telecentric manipulating mechanism includes a movable platform, a static platform, and a plurality of telescopic units. Two ends of each telescopic unit are respectively rotatably connected to the movable platform and the static platform, and the plurality of telescopic units are configured to move forward and backward in a cooperation manner to drive the movable platform to move relative to the static platform. The executing mechanism is arranged on the movable platform, and the surgical instrument has a preset telecentric fixed point, and deflection of the movable platform is capable of driving the surgical instrument to swing around the telecentric fixed point.

### BRIEF DESCRIPTION OF DRAWINGS

FIG. 1 is a schematic view of an executing mechanism according to an embodiment of the present disclosure, where the end of the outer sheath close to the pair of swing members is partially cut away in order to show a pair of push rods within the outer sheath.
FIG. 2 is an exploded view of the executing mechanism in FIG. 1.
FIG. 3 is an exploded view of the executing mechanism omitting the housing and the surgical instrument, which mainly shows an exploded push rod driving assembly.
FIG. 4 is a schematic view of the exploded structure shown in FIG. 3 further omitting the base, where the junction of the push rod limiting assembly and the assembly transition plate is further exploded.
FIG. 5 is an exploded view of the outer sheath limiting assembly and the push rod limiting assembly.
FIG. 6 is a schematic view of a sliding sleeve.
FIG. 7 is another perspective view of the sliding sleeve shown in FIG. 6.
FIG. 8 is a sectional view of the sliding sleeve shown in FIG. 6.
FIG. 9 is an exploded view of the assembly of the push rod limiting assembly and the surgical instrument.
FIG. 10 is a schematic view of a base.
FIG. 11 is a schematic view of the assembly of the mounting-dismounting control assembly on the base, where the shift block and the shift block connector are in the locked position.
FIG. 12 is a schematic view of the assembly of the mounting-dismounting control assembly on the base, where the shift block and the shift block connector are in the detachable position.
FIG. 13 is a schematic view of the executing mechanism omitting the housing and the push rod driving assembly on one side.
FIG. 14 is a schematic view of an executing mechanism according to another embodiment of the present disclosure, where the housing is omitted in order to show the internal structure of the housing.
FIG. 15 is a schematic view of the structure shown in FIG. 14 omitting the surgical instrument.
FIG. 16 is a schematic view of a surgical robot according to an embodiment of the present disclosure, where multiple groups of executing mechanisms shown in FIG. 1 are assembled.
100, executing mechanism; 200, telecentric manipulating mechanism; 300, presurgical positioning mechanism; 400, frame; 500, mount;
11, mounting-dismounting control assembly; 111, joint unlocking member; 1111, fixing hole; 1112, insertion portion; 1113, sleeving portion; 112, shift block; 113, shift block connector; 1131, accommodating hole; 1132, connecting end; 12, surgical instrument; 121, outer sheath; 1211, annular limiting groove; 1212, connection groove; 122, push rod; 1221, locking groove; 123, swing member; 13, outer sheath limiting assembly; 131, limiting element; 1311, insertion hole; 1312, outer sheath limiting hole; 132, first spring; 133, blocking piece; 134, detection element; 14, push rod limiting assembly; 141, sliding sleeve; 1411, locking section; 1412, unlocking section; 1413, guiding groove; 1414, blocking portion; 142, push rod locking sleeve; 1421, guiding rib; 1422, locking hole; 143, push rod locking member; 144, second spring; 15, push rod driving assembly; 151, linear motor; 152, mounting seat; 153, loading seat; 154, guiding member; 1541, bushing; 1542, guiding shaft; 155, assembly transition plate; 16, base; 160, base body; 1600, inner cavity; 161, support portion; 162, slideway; 1621, inclined surface; 1622, protrusion; 163, slide hole; 164, linked protrusion; 165, support protrusion; 17, housing; 170, through hole.

### DESCRIPTION OF EMBODIMENTS

The technical solutions according to the embodiments of the present disclosure will be described clearly and fully in combination with the drawings according to the embodiments of the present disclosure. Obviously, the described embodiments are not all embodiments of the present disclosure, but only part of the embodiments of the present disclosure. Based on the disclosed embodiments, all other embodiments obtained by those skilled in the art without creative work fall into the scope of this disclosure.

It should be noted that, when a component is "mounted" with another component, it may be directly mounted to another component or may be indirectly mounted to another component through a further component. When a component is "provided" on another component, it may be directly provided on another component or may be provided on another component through a further component. When a component is "fixed" on another component, it may be directly fixed on another component or may be fixed on another component through a further component.

Unless otherwise defined, all technical and scientific terms used herein have the same meaning as commonly understood by a person skilled in the art. The terms in the description of the present disclosure are used to describe specific embodiments, and not to limit the present disclosure. The term "and/or" used herein includes one or more of the listed options in any combinations, or the combination of all of the listed options.

The quick insertion-and-removal device according to the first aspect of the present disclosure is adapted to the mounting and dismounting of a surgical instrument of a surgical robot. In order to better understand the structure of the quick insertion-and-removal device, an example of the surgical instrument adapted to the quick insertion-and-removal device will be first described below.

This kind of surgical instrument is adapted to be installed on the front end (also distal end) of the surgical robot for assisting a minimally invasive surgery. In the minimally invasive surgery using this surgical robot, only a tiny wound is opened on the patient's body, and the end of the surgical instrument is inserted into the patient's body via the tiny wound until reaching the lesion. In order to protect the wound from being pulled during the procedure, the surgical instrument needs to swing with the wound as the telecentric fixed point. In other words, during the swinging process of the surgical instrument, the surgical instrument has no displacement and remains stationary at the wound all the time. In this kind of surgical robot, the range of motion, flexibility and control precision of a surgical tool of the surgical instrument that is configured to reach the lesion are critical. However, due to the tiny wound, the size of the surgical instrument should not be too large. Therefore, the driving structure for the surgical tool of the surgical instrument is limited in design.

For some surgical robots, a steel cable is used as the driving member for the surgical tool, where the steel cable is configured to pull and thus drive the surgical tool to deflect or open and close. However, after the steel cable pulls and drives the surgical tool for multiple times, it will become elongated and deformed to a certain extent. After that, the motion relationship between the pulling distance of the steel cable and the swing range of the surgical tool changes, resulting the control accuracy decreased. In this case, within the same range of motion for the surgical tool, the steel cable needs to be pulled a greater distance, causing a low transmission efficiency and great creepage for the surgical instrument.

In order to avoid the disadvantages of the steel cable as the driving structure, a rigid rod can be alternatively used as the driving member for the surgical instrument to drive the surgical tool to perform the surgical operations. No matter which driving structure is used, it is always necessary to replace the surgical tool during the procedure. Therefore, a quick mounting and dismounting structure for the surgical instrument is also crucial when designing this kind of surgical robot. In the case where the steel cable is used as the driving structure, for convenience of replacing the surgical instrument to meet the requirements of different procedures, a discardable end assembly is generally arranged at the distal end of the surgical instrument away from the operating end. However, when the rigid rod is used as the driving member, the mounting and dismounting structure of the surgical instrument also needs to be redesigned.

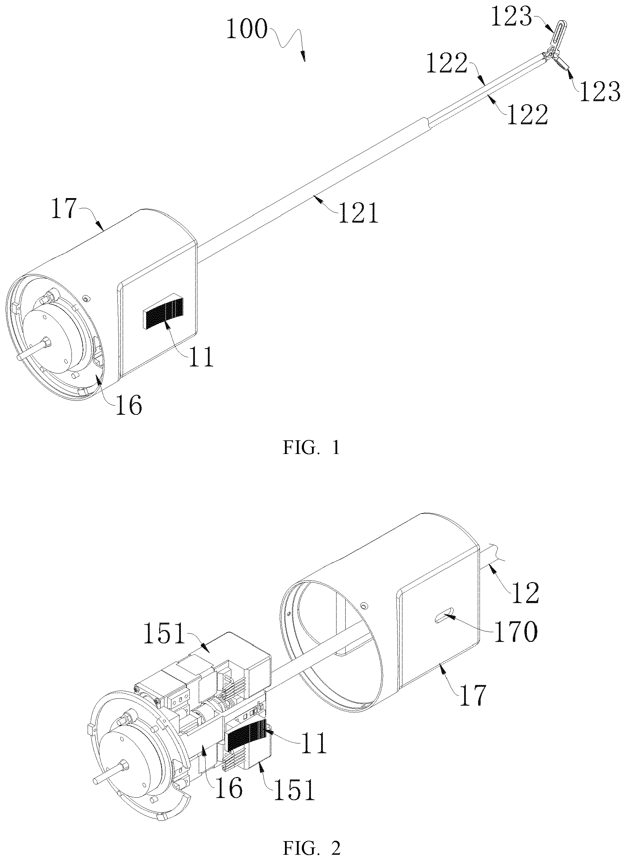

With reference to shown in FIG. 1, the surgical instrument with the rigid rod as the driving structure for the surgical tool at least includes an outer sheath 121, a push rod 122 slidably arranged in the outer sheath 121, and a pair of swing members 123 drivably connected with the push rod 122 at one end. The pair of swing members 123 and related structures together form the surgical tool for performing the preset surgical operations.

As mentioned above, in order to avoid the low transmission accuracy and creepage caused by using the cable, the push rod 122 is used as the driving member for the surgical tool. In order to increase the swing range of the single swing member 123 of the surgical tool, thereby ensuring the flexibility of the surgical tool, the surgical instrument 12 of the present disclosure includes two push rods 122, each of which is configured to drive a respective swing member 123. It can be understood that the quick insertion-and-removal device according to the present disclosure is suitable for either a single push rod 122 or two push rods 122. In other words, the quick insertion-and-removal device according to the present disclosure is suitable for the surgical instrument 12 having the outer sheath 121 and the push rod(s) 122, for quickly unlocking the outer sheath 121 and the push rod(s) 122 when replacing the surgical instrument.

Referring to FIG. 2, FIG. 4 and FIG. 5, the quick insertion-and-removal device includes a base 16, an outer sheath limiting assembly 13, a push rod limiting assembly 14 and a mounting-dismounting control assembly 11, wherein the surgical instrument 12 is slidably connected to the base 16. In disassembly, the surgical instrument 12 can slide relative to the base 16 as a whole to release the constraint and assembly therebetween, while in use, the push rod 122 in the surgical instrument 12 can slide within the outer sheath 121 relative to the base 16 to drive the surgical tool to move.

The outer sheath limiting assembly 13 is provided on the base 16 and includes a limiting element 131 that can move along a direction perpendicular to the sliding direction of the surgical instrument 12 relative to the base 16, wherein the movement of the limiting element 131 relative to the base 16 can lock or unlock the outer sheath 121. Similarly, the push rod limiting assembly 14 is also provided on the base 16 and includes a sliding sleeve 141 which can move relative to the base to lock or unlock the push rod 122.

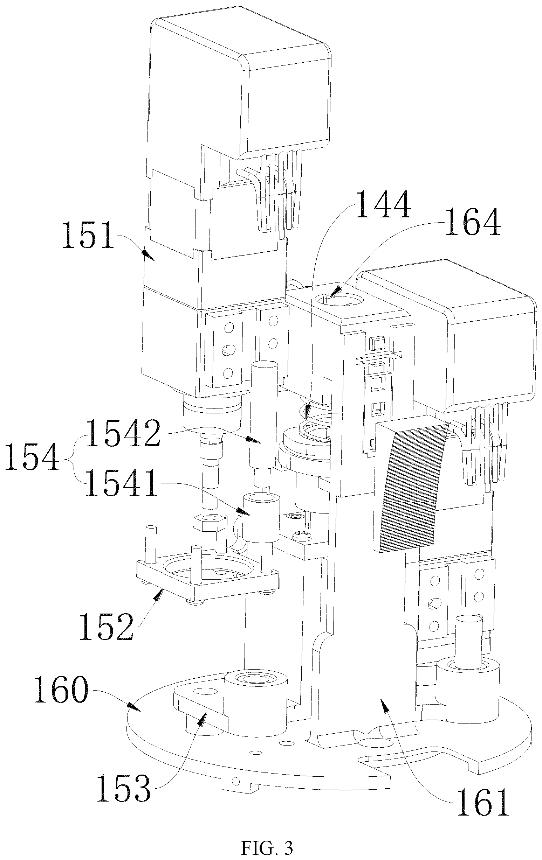

Referring to FIG. 3, FIG. 5 and FIG. 11, in operation of the surgical robot, the surgical instrument needs to rotate with the base 16 as a whole. In order to realize the circumferential linkage between the outer sheath 121 and the base 16, the base 16 is provided with a linked protrusion 164 extending radially within a bore for receiving the outer sheath 121, and correspondingly, the outer sheath 121 is provided with a connection groove 1212 for the linked protrusion 164 being accommodated and sliding therein. Therefore, when disassembling the outer sheath 121, the user only needs to unlock the axial positioning. When the outer sheath 121 is pulled out of the base 16, the linked protrusion 164 slides in the connection groove 1212. When the outer sheath 121 is exactly inserted within the base 16, the linked protrusion 164 and the connection groove 1212 engage with each other like in a key connection, so that the base 16 and the outer sheath 121 can be circumferentially linked.

The mounting-dismounting control assembly 11 is used to unlock the outer sheath 121 and the push rod 122 simultaneously, and includes a joint unlocking member 111 that can move relative to the base 16. When the joint unlocking member 111 slides relative to the base 16, it can drive the sliding sleeve 141 of the push rod limiting assembly 14 to move together, so that the sliding sleeve 141 slides relative to the base 16 to the position where the push rod 122 can be unlocked. When the sliding sleeve 141 slides to the position of unlocking the push rod 122, the joint unlocking member 111 engages with the limiting element 131 of the outer sheath limiting assembly 13 to unlock the outer sheath 121.

It can be understood that unlocking the push rod 122 requires the movement of the sliding sleeve 141 relative to the base 16, while unlocking the outer sheath 121 requires the movement of the limiting element 131 relative to the base 16. The joint unlocking member 111 moving relative to the base 16 can realize the above two movements simultaneously. Therefore, the user can unlock the outer sheath 121 and the push rod 122 by separately operating the joint unlocking member 111, improving the unlocking efficiency of the surgical instrument 12, with a simple operation.

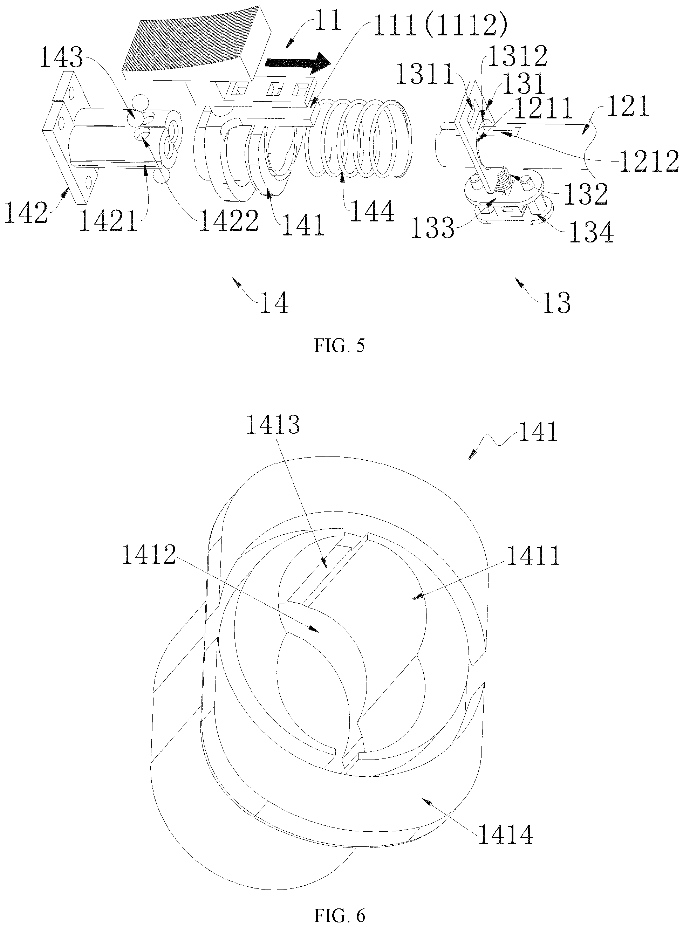

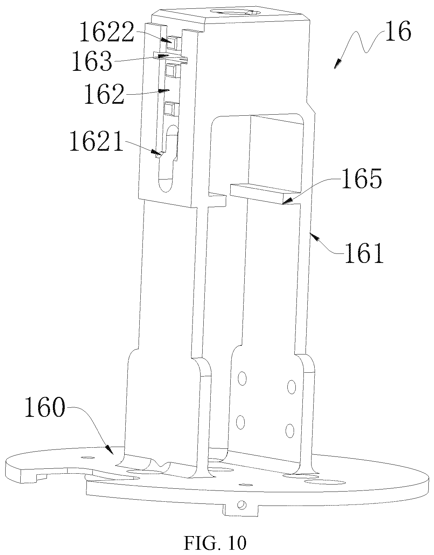

Referring to FIG. 5 and FIG. 10, the base 16 is opened with a slide hole 163 extending perpendicular to the sliding direction of the outer sheath 121, and the limiting element 131 is slidably received within the slide hole 163. The limiting element 131 is provided with an outer sheath limiting hole 1312 for engaging with the outer sheath 121, which can be configured as a complete or local hole for engaging with the outer wall of the outer sheath 121 to limit the sliding of the outer sheath 121. In the embodiment shown in FIG. 5, the outer sheath limiting hole 1312 is an irregular hole around the outer sheath 121. When the limiting element 131 slides within the slide hole 163, different sections of the outer sheath limiting hole 1312 engage with the outer wall of the outer sheath 121. When the outer sheath limiting hole 1312 does not interfere with the outer sheath 121, the outer sheath 121 is unlocked.

Further, in order to make the limiting effect of the outer sheath limiting hole 1312 more reliable, in one embodiment, an annular limiting groove 1211 is defined in the outer wall of the outer sheath 121 at a predetermined axial position, and part of the hole wall of the outer sheath limiting hole 1312 can extend into the annular limiting groove 1211. As mentioned above, in order to enable the outer sheath 121 to be linked with the base 16 in the circumferential direction, they are circumferentially engaged with each other through the connection groove 1212 and the linked protrusion 164. Therefore, in the embodiment with the annular limiting groove 1211, the connection groove 1212 extends through the annular limiting groove 1211, and preferably until the end of the outer sheath 121 (as shown in FIG. 5).

As can be seen from the foregoing, unlocking the outer sheath 121 requires the sliding of the limiting element 131 within the slide hole 163. Therefore, in one embodiment, when the joint unlocking member 111 moves to engage with the limiting element 131, the joint unlocking member 111 unlocks the outer sheath 121 by driving the limiting element 131 to slide.

Continuing to refer to FIG. 5, in the illustrated embodiment, the limiting element 131 is opened with an insertion hole 1311 for receiving the joint unlocking member 111. As the joint unlocking member 111 slides, it gradually engages with the insertion hole 1311, while the engagement of the two does not directly result in the movement of the limiting element 131. An additional external force applied to the joint unlocking member 111 is required to drive the limiting element 131 to slide to the position of unlocking the outer sheath 121. In other embodiments, the outer sheath 121 can be unlocked by using other methods. For example, in an alternative, the insertion hole 1311 in the limiting element 131 can be elongated in the direction in which the joint unlocking member 111 moves and engages therewith, and be configured as a diameter varying hole. When engaging the joint unlocking member 111 into the insertion hole 1311, due to the diameter change of the insertion hole 1311, the limiting element 131 gradually slides along with the joint unlocking member 111 to the position of unlocking the outer sheath 121.

Referring to FIGS. 10 to 12, the mounting-dismounting control assembly 11 can further include a shift block connector 113, one side of which is fixedly connected to the joint unlocking member 111, and the other side extends along the motion direction of the joint unlocking member 111 as a slidable portion.

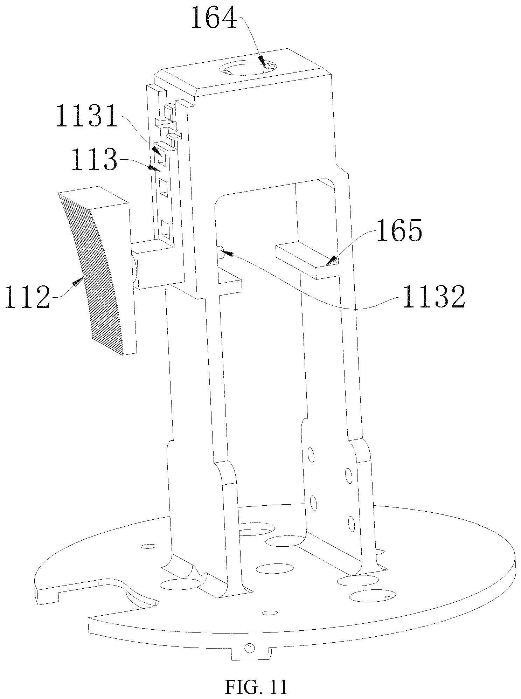

Specifically, referring to the orientation shown in FIG. 11, the lower side of the shift block connector 113 is provided with a connecting end 1132 extending toward the interior of the base 16. The connecting end 1132 is used to connect to a fixing hole 111 defined in the joint unlocking member 111 as shown in FIG. 5 which shows the relative positional relationship therebetween. The slidable portion (not marked with reference sign in the figure) is slidably connected to the base 16. When the user drives the shift block connector 113 to slide relative to the base 16, the connecting end 1132 of the shift block connector 113 can drive the joint unlocking member 111 to move together, while the slidable portion can make the movement of the shift block connector 113 more stable through its engagement with the base 16.

Referring to FIG. 10, a slideway 162 is opened in one side of the base 16 for slidably connecting with the slidable portion of the shift block connector 113, and at least one side of the shift block connector 113 can be in a sliding fit with at least one wall of the slideway 162, so that the slideway 162 can guide the sliding shift block connector 113.

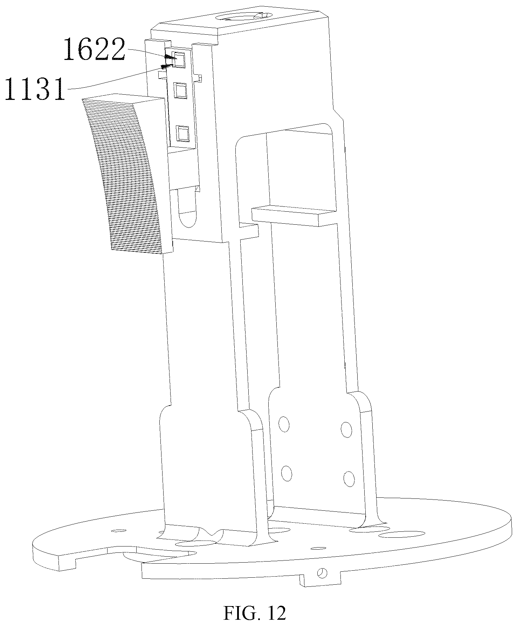

Further, as shown in FIG. 11 and FIG. 12, a protrusion 1622 is provided on the bottom wall of the slideway 162, and the side surface of the protrusion 1622 facing away from the bottom wall of the slideway 162 is the top surface of the protrusion 1622. The slidable portion of the shift block connector 113 is correspondingly provided with an accommodating hole 1131 for receiving the protrusion 1622 so that the shift block connector 113 can reach the bottom wall of the slideway 162.

As shown in FIG. 11, before the shift block connector 113 drives the joint unlocking member 111 to slide to the position of unlocking the outer sheath 121, the sliding shift block connector 113 is supported on the top surface of the protrusion 1622. As shown in FIG. 12, when the joint unlocking member 111 slides to the position where it engages with the limiting element 131 and the outer sheath 121 can be unlocked, the accommodating hole 1131 surrounds the protrusion 1622, and the shift block connector 113 can reach the base 16 due to the engagement of the accommodating hole 1131 with the protrusion 1622, thereby driving the limiting element 131 to slide in the slide hole 163 and unlocking the outer sheath 121.

Referring to FIG. 10, the bottom of the slideway 162 is further provided with an inclined surface 1621. When the shift block connector 113 moves from the position shown in FIG. 11 to the position shown in FIG. 12, the shift block connector 113 slides on the inclined surface 1621, so that the shift block connector 113 gradually approaches the side of the base 16.

Along the extension direction of the slideway 162 (or along the sliding direction of the shift block connector 113 relative to the base 16), a plurality of protrusions 1622 are provided at intervals, so that the top surfaces of the plurality of protrusions 1622 can support the slidable portion of the shift block connector 113 more stably. It can be understood that the slide hole 163 is disposed between two adjacent protrusions 1622. Corresponding to the plurality of protrusions 1622 arranged at intervals, the number and locations of the accommodating holes 1131 are defined corresponding to the number and locations of the protrusions 1622.

As shown in FIG. 11, the shift block connector 113 can be further connected with a shift block 112, which protrudes through the through hole 170 defined in the housing 17 of the surgical instrument as shown in FIG. 2. This through hole 170 can be configured as an elongated hole extending along the sliding direction of the shift block connector 113. Therefore, the user can control the movement of the shift block connector 113 via the shift block 112 outside the housing 17, thereby driving the joint unlocking member 111 to move. Further, anti-slip strips are provided on the outer surface of the shift block 112, and this surface is formed as a curved slope, which facilitates the user to apply an external force on the shift block 112 to perform unlocking.

Referring back to FIG. 5, the outer sheath limiting assembly 13 further includes a detection element 134, which is installed within the sliding path of the limiting element 131 to detect the stop position of the limiting element 131 inside the hole 163 as shown in FIG. 10. The detection element 134 can be configured as a device which can give a intuitive feedback signal such as a photoelectric switch, so that when the limiting element 131 slides to the position of unlocking the outer sheath 121, the photoelectric switch as the detection element 134 can be triggered to emit light signal to remind the user that the outer sheath 121 has been unlocked, and the surgical instrument 12 can be removed and replaced as a whole.

Further, the outer sheath limiting assembly 13 can include a first spring 132. The spring force from the first spring 132 acts on the limiting element 131 to keep the outer sheath 121 locked. As mentioned above, when unlocking the outer sheath 121, the external force applied to the limiting element 131 pushes the limiting element 131 close to the detection element 134. Therefore, in the illustrated embodiment, the spring force from the first spring 132 pushes against the limiting element 131 away from the detection element 134.

In one embodiment, for convenience of the installation of the first spring 132, the outer sheath limiting assembly 13 further includes a blocking piece 133. The blocking piece 133 cooperates with the limiting element 131 to constrain the first spring 132 therebetween in a compressed state. The blocking piece 133 is fixed relative to the base 16, so that the first spring 132 in the compressed state can push against the limiting element 131 away from the detection element 134. Alternatively, in other embodiments, the first spring 132 can be in a stretched state. In such case, by changing the relative position between the first spring 132 and the limiting element 131, the same effect can be achieved. Further alternatively, the blocking piece 133 can be removed, and an appropriate portion on the base 16 can be used as a constraining portion similar to the first spring 132.

As shown in FIG. 5, the limiting element 131 is provided with a first linked engagement portion, and the joint unlocking member 111 is provided with a second linked engagement portion. The first linked engagement portion and the second linked engagement portion can engage with each other to realize the engagement between the joint unlocking member 111 and the limiting element 131. It can be seen from the foregoing that the first linked engagement portion can be configured as the insertion hole 1311 opened in the limiting element 131, and accordingly the second linked engagement portion can be configured as an insertion portion 1112 on the joint unlocking member 111. It can be appreciated that, in order to insert the insertion portion 1112 into the insertion hole 1311 in the limiting element 131 more accurately and smoothly, the insertion portion 1112 can be configured as a wedge-shaped structure. The front end of the wedge-shaped structure is narrow and the rear end gradually widens, so that the insertion portion 1112 can be smoothly guided into the insertion hole 1311.

When the shift block 112 slides along the solid arrow direction shown in FIG. 5 with the shift block connector 113 and the joint unlocking member 111, the insertion portion 1112 can be inserted into the insertion hole 1311 to realize the engagement between the joint unlocking member 111 and the limiting element 131. The first linked engagement portion and the second linked engagement portion can alternatively use other configurations, which will not be described here. The configurations which can realize the engagement between the joint unlocking member 111 and the limiting element 131 all belong to the equivalent alternatives of the insertion portion 1112 and the insertion hole 1311.

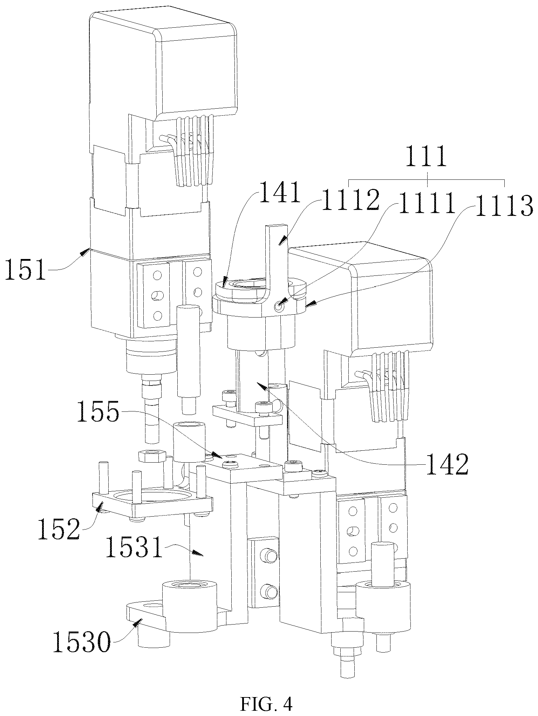

Referring to FIG. 4 and FIG. 5, the joint unlocking member 111 further includes a sleeving portion 1113 for surrounding the sliding sleeve 141, and correspondingly, the sliding sleeve 141 includes a blocking portion 1414 engageble with the sleeving portion 1113. The radial size of the blocking portion 1414 is larger than the diameter of the inner hole of the sleeving portion 1113, so that the joint unlocking member 111 can drive the sliding sleeve 141 to slide along the solid arrow direction shown in FIG. 5 through the sleeving portion 1113. In order to enable the joint unlocking member 111 to drive the limiting element 131 to move radially relative to the sliding sleeve 141 after sliding to engage with the limiting element 131, the inner diameter of the sleeving portion 1113 is slightly greater than the remaining portion of the sliding sleeve 141 except for the blocking portion. The engagement between the sleeving portion 1113 and the blocking portion 1414 only limits the axial linkage between the joint unlocking member 111 and the sliding sleeve 141, without affecting the radial movement of the joint unlocking member 111 relative to the sliding sleeve 141 within a preset range. In this way, when the joint unlocking member 111 moves together with the sliding sleeve to engage with the limiting element 131, the joint unlocking member 111 can move radially relative to the sliding sleeve 141, thereby driving the limiting element 131 engaged with the joint unlocking member 111 to move to unlock the outer sheath 121.

Referring to FIGS. 3 and 5, the push rod limiting assembly 14 further includes a second spring 144 disposed between the sliding sleeve 141 and the base 16. The spring force from the second spring 144 drives the sliding sleeve 141 to tend to move away from the limiting element 131, that is, the spring force from the second spring 144 is directed so that the sliding sleeve 141 can be retained at the position of locking the push rod 122 until the pushing force applied on the shift block 112 counteracts the spring force from the second spring 144.

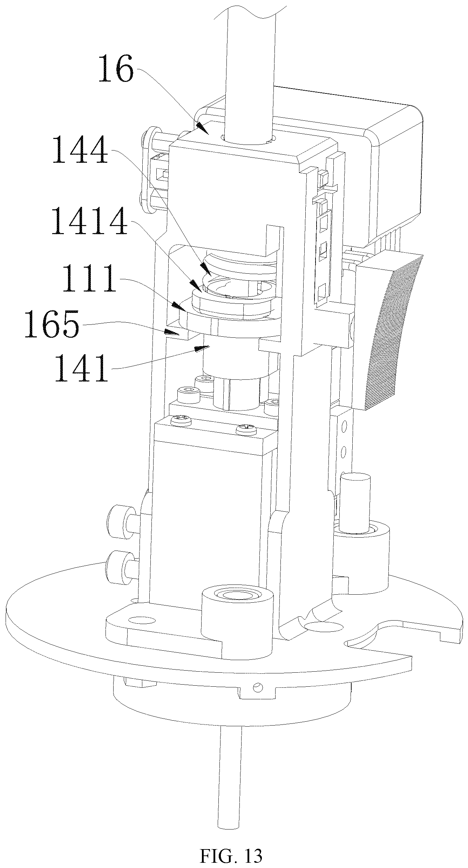

As shown in FIG. 10, FIG. 11 and FIG. 13, the spring force from the second spring 144 is directed so that the sliding sleeve 141 tends to move downward (referring to the orientation shown in FIG. 13). In order to prevent the sliding sleeve 141 and the joint unlocking member 111 from moving downward over the preset travel, the base 16 is provided with a support protrusion 165. As mentioned above, the joint unlocking member 111 is engaged with the blocking portion 1414 on the sliding sleeve 141. Therefore, the lower end of the joint unlocking member 111 would be supported by the support protrusion 165 on the base 16 and cannot continue to move downward. The spring force from the second spring 144 finally acts on the joint unlocking member 111 through the blocking portion 1414 and flexibly presses it against the support protrusion 165.

Referring to FIG. 5 to FIG. 9, the push rod limiting assembly 14 is used to detachably lock the push rod 122 thereon. Therefore, in addition to the aforementioned sliding sleeve 141, the push rod limiting assembly 14 can further include a push rod locking sleeve 142 and a push rod locking member 143. The end of the push rod 122 away from the surgical tool is inserted in the push rod locking sleeve 142, and the push rod locking sleeve 142 is slidably received in the sliding sleeve 141. The push rod locking member 143 is restrained inside the sliding sleeve 141, and can be pushed between the push rod locking sleeve 142 and the push rod 122 by the sliding sleeve 141 to lock the push rod 122. When the sliding sleeve 141 slides with the joint unlocking member 111 relative to the push rod locking sleeve 142, the sliding sleeve 141 releases the push rod locking member 143, thereby unlocking the push rod 122 relative to the push rod locking sleeve 142.

In the illustrated embodiment, the push rod locking member 143 is configured as a spherical member independent of the push rod locking sleeve 142 and the push rod 122. In other feasible embodiments, the push rod locking member 143 can use other forms, and/or be configured as a member installed on the push rod locking sleeve 142 or the push rod 122, as long as it can achieve the above-mentioned locking and unlocking effects.

In order to balance the forces on two sides of the axis of the push rod 122, each push rod 122 can correspond to one or more pairs of push rod locking members 143, and the two push rod locking members 143 in each pair are axially symmetrical to each other with respect to the axis of the push rod 122.

As shown in FIG. 5, a locking hole 1422 is opened in the push rod locking sleeve 142, and the push rod locking member 143 as a spherical member is movably arranged in the locking hole 1422 so that it can roll and slide in the locking hole 1422. In one embodiment, the locking hole 1422 is configured as a diameter varying hole, so that the push rod locking member 143 can expose less than half of its volume from the opening of the locking hole 1422 near the side where the push rod 122 is inserted, so as to realize the locking of the push rod 122; on the other hand, the push rod locking member 143 can easily move toward the inner wall of the sliding sleeve 141 from the other side of the locking hole 1422 with a slightly larger opening, so that when the sliding sleeve 141 slides to the position of unlocking the push rod 122, the push rod locking member 143 can respond quickly to unlock the push rod 122.

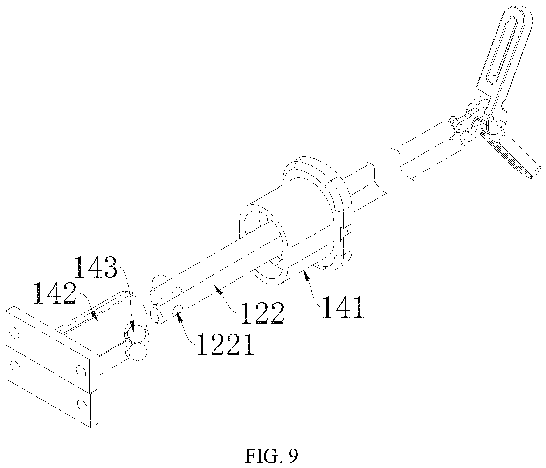

Referring to FIG. 9, the push rod 122 is provided with a locking groove 1221 into which a part of the push rod locking member 143 can be embedded. As can be seen from the aforementioned, when the sliding sleeve 141 locks the push rod 122, it can push the push rod locking member 143 toward the push rod 122. In the embodiment provided with the locking groove 1221, the push rod locking member 143 being pushed toward the push rod 122 can be partially embedded in the locking groove 1221, so as to lock the push rod 122 more reliably.

Referring to FIGS. 6 to 8, the sliding sleeve 141 includes a locking section 1411 and an unlocking section 1412 arranged along the sliding direction of the sliding sleeve 141, in which the inner diameter of the sliding sleeve 141 corresponding to the unlocking section 1412 is greater than the inner diameter of the sliding sleeve 141 corresponding to the locking section 1411. When the locking section 1411 corresponds to the push rod locking sleeve 142, the inner wall of the sliding sleeve 141 corresponding to the locking section 1411 pushes the push rod locking member 143 into the locking groove 1221 in the push rod 122. When the unlocking section 1412 corresponds to the push rod locking sleeve 142, there is a gap between the inner wall of the sliding sleeve 141 and the outer wall of the push rod locking sleeve 142, and the push rod locking member 143 can move within the locking hole 1422 towards the opening of the locking hole 1422 away from the locking groove 1221, so that the push rod 122 can be unlocked due to the disengagement of the push rod locking member 143 from the locking groove 1221.

In one embodiment, the inner wall of the sliding sleeve 141 corresponding to the locking section 1411 can generally slide along and fit with the outer wall of the push rod locking sleeve 142, thereby ensuring that the push rod locking member 143 can be reliably pressed into the locking groove 1221.

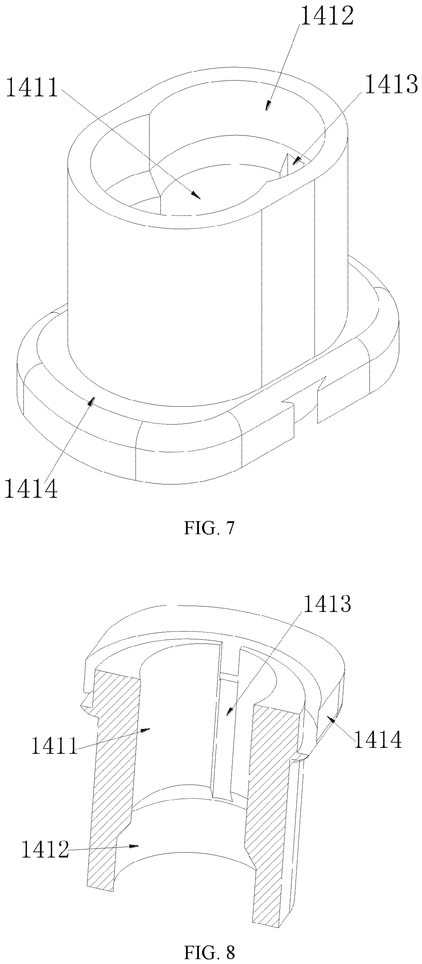

Further, as shown in FIG. 5 and FIG. 8, in order to restrict the relative sliding between the push rod locking sleeve 142 and the sliding sleeve 141 in only one direction (i.e., the axial direction of the push rod 122), one of the two is provided with a guiding rib 1421, and the other is provided with a guiding groove 1413 in a sliding fit with the guiding rib 1421. In the illustrated embodiment, the guiding rib 1421 protrudes from the outer wall of the push rod locking sleeve 142, and the guiding groove 1413 is opened in the sliding sleeve 141 and extends through the locking section 1411 and the unlocking section 1412.

It can be seen from the foregoing description that the number of push rods 122 does not affect the function of the quick insertion-and-removal device. Both single push rod 122 and two push rods 122 can be locked and unlocked through the push rod limiting assembly 14 described above. Depending on the number of push rods 122, those skilled in the art can adaptively adjust the outer shape of the push rod locking sleeve 142 and the inner shape of the sliding sleeve 141, so as to adapt to the configuration with a single push rod or two push rods.

The second aspect according to the present disclosure further provides an executing mechanism 100, which includes the quick insertion-and-removal device according to any one of the above-mentioned embodiments, and a surgical instrument 12. Referring to FIG. 10, the base 16 includes a base body 160 and a support portion 161, and the surgical instrument 12 and most of the quick insertion-and-removal device are mounted on the support portion 161.

Referring to FIGS. 2 to 4, the base 16 is provided with a push rod driving assembly 15 for driving the push rod 122 to slide back and forth within the outer sheath 121. When two push rods 122 are provided, correspondingly, two groups of push rod driving assemblies 15 are provided. Taking one group of push rod driving assembly 15 as an example, it can include a linear driving device installed on the support portion 161 of the base 16, and a loading seat 153. The loading seat 153 is connected to the output end of the motion of the linear driving device, so as to drive the push rod 122 to slide back and forth in the axial direction.

Specifically, the linear driving device can be selected as a linear motor 151 or other similar devices. When the linear driving device is selected as the linear motor 151, the push rod driving assembly 15 can further include a guiding member 154. Generally, the linear motor 151 includes a group of common stepping motor/servo motor and a screw-nut assembly. The precise linear motor 151 can use a ball screw-nut assembly to convert the motion, which outputs rotational motion to drive the screw of the ball screw-nut assembly to rotate, and based on the engagement between the screw and the nut, then the nut moves linearly along the axial direction of the screw. The linear motor 151 is fixedly installed on the base 16 through a mounting seat 152.

The guiding member 154 is used to limit the rotational movement of the loading seat 153 so that it can only move linearly with the screw-nut assembly. One end of the push rod locking sleeve 142 of the push rod limiting assembly 14 is provided with a connecting flange (not marked with reference sign in the figure, the structure having a connecting hole is the connecting flange), and can be directly or indirectly fixedly connected with the loading seat 153 through the connecting flange. In order to simplify the assembly between the push rod locking sleeve 142 and the loading seat 153, the push rod driving assembly 15 can further include an assembly transition plate 155, so that the assembly position of the push rod locking sleeve 142 and the loading seat 155 can be flexibly adjusted through the assembly transition plate 155.

Continuing to refer to FIG. 3 and FIG. 4, the guiding member 154 includes a bushing 1541 embedded and fixed in the loading seat 153, and a guiding shaft 1542 fixedly installed on the base body 160. The guiding shaft 1542 is fixed. When the linear motor 151 drives the loading seat 153 to slide, the bushing 1541 is in a sliding fit with the guiding shaft 1542 so that the loading seat 153 can only move linearly in one direction. In other embodiments, the guiding member 154 can be replaced with a structure like a guiding rail, provided that it can achieve the above-mentioned limiting and guiding effect. The guiding member 154 can use various other alternatives, which would not be described in detail here.

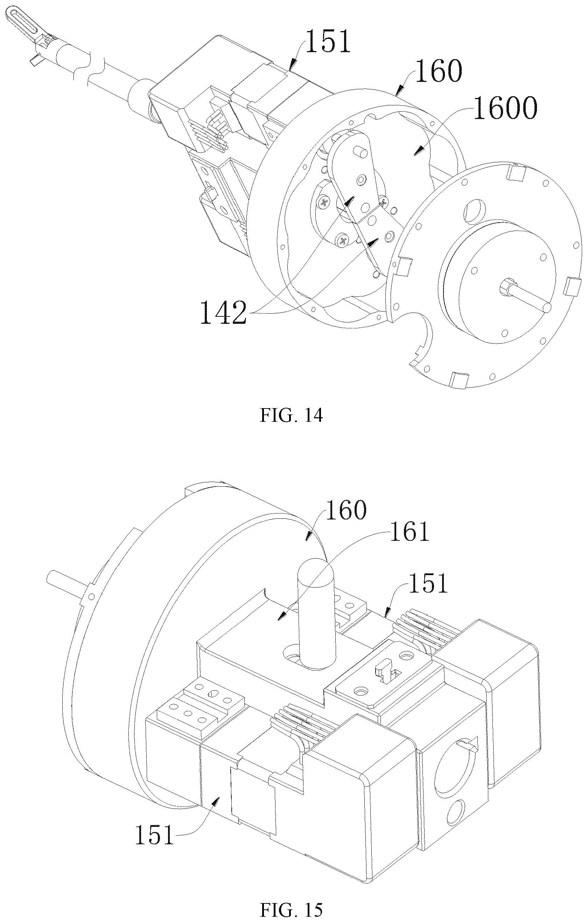

Referring to FIG. 14 and FIG. 15, another embodiment of the executing mechanism is shown in the figures, which mainly differs from the previous embodiment in that an inner cavity 1600 is defined in the base body 160 of the base 16, and the linear motor 151 is directly installed on one side of the base body 160; the inner cavity 1600 of the base body 160 communicates with the space inside the support portion 161, and one end of the push rod locking sleeve 142 can extend into the inner cavity 1600 and be connected with the output end of the motion of the linear motor 151 within the inner cavity 1600. In this embodiment, since the linear motor 151 is installed directly on one side of the base body 160, the installation accuracy of the linear motor 151 will be greatly improved, thereby improving the motion accuracy of the push rod locking sleeve 142, and thus the motion accuracy of the push rod installed in the push rod locking sleeve 142.

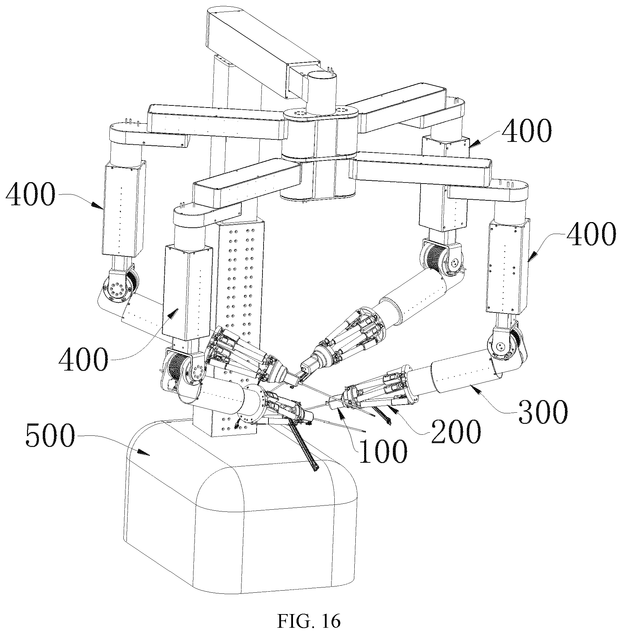

Referring to FIG. 16, the third aspect according to the present disclosure further provides a surgical robot, including the executing mechanisms 100 according to the aforementioned embodiment, telecentric manipulating mechanisms 200, presurgical positioning mechanisms 300, frames 400 and a mount 500. There are multiple groups of frames 400 disposed on the mount 500, and each frame 400 is correspondingly provided with a group of presurgical positioning mechanism 300, a telecentric manipulating mechanism 200 and an executing mechanism 100.

A possible implementation of the telecentric manipulating mechanism 200 includes a movable platform, a static platform and a plurality of telescopic units. Two ends of each telescopic unit are rotatably connected to the movable platform and the static platform. The plurality of telescopic units moves forward and backward in a cooperation manner to drive the movable platform to move relative to the static platform.

The executing mechanism 100 is arranged on the movable platform. The surgical instrument 12 has a preset telecentric fixed point, and the deflection of the movable platform can drive the surgical instrument 12 to swing around the telecentric fixed point. During minimally invasive surgery, the executing mechanism 100 is manipulated by the presurgical positioning mechanism 300 so that the telecentric fixed point on the surgical instrument 12 coincides with the tiny wound on the patient's body. Therefore, in the subsequent surgical, as the surgical instrument 12 swings about the telecentric fixed point, the surgical instrument 12 will not pull the wound.

The technical features of the above embodiments can be arbitrarily combined, and not all possible combinations of the technical features of the above embodiments have been described for the sake of brevity of description. However, as long as there is no contradiction in the combination of these technical characteristics, such combination should be regarded as falling into the scope of this specification.

The skilled person in the art should recognize that the above embodiments are only used to illustrate the present disclosure, rather than as limitations to the present disclosure. Provided within the scope of the spirit of the present disclosure, appropriate changes and modifications made to the above embodiments all fall within the scope of protection of the present disclosure.

## Claims

1. A quick insertion-and-removal device for a surgical instrument, comprising:
a base for slidably connecting the surgical instrument;
an outer sheath limiting assembly provided on the base and comprising a limiting element, the limiting element is movable relative to the base in a direction perpendicular to a sliding direction of the surgical instrument and used for locking or unlocking an outer sheath of the surgical instrument;
a push rod limiting assembly provided on the base and comprising a sliding sleeve which is movable relative to the base to lock or unlock a push rod of the surgical instrument; and
a mounting-dismounting control assembly comprising a joint unlocking member, the joint unlocking member is movable relative to the base and connected to the sliding sleeve and capable of driving the sliding sleeve to move relative to the base to a position of unlocking the push rod, where the joint unlocking member engages with the limiting element and is capable of unlocking the outer sheath.

2. The quick insertion-and-removal device for the surgical instrument according to claim 1, wherein the base has a slide hole extending perpendicular to a sliding direction of the outer sheath, the limiting element is slidably connected to the slide hole and opened with an outer sheath limiting hole and is capable of locking or unlocking the outer sheath of the surgical instrument through the outer sheath limiting hole.

3. The quick insertion-and-removal device for the surgical instrument according to claim 2, wherein when the joint unlocking member moves to a position of being engaged with the limiting element, the joint unlocking member is capable of driving the limiting element to slide in the slide hole to unlock the outer sheath.

4. The quick insertion-and-removal device for the surgical instrument according to claim 3, wherein the mounting-dismounting control assembly further comprises a shift block connector, one side of which is fixedly connected to the joint unlocking member, and the other side extends along a motion direction of the joint unlocking member to form as a slidable portion.

5. The quick insertion-and-removal device for the surgical instrument according to claim 4, wherein a protrusion is provided on one side of the base; an accommodating hole is defined in the slidable portion for accommodating the protrusion; and the shift block connector is slidable relative to the base on a surface where a top face of the protrusion is located, and when the shift block connector slides with the joint unlocking member to the position where the joint unlocking member is engaged with the limiting element, the accommodating hole accommodates the protrusion as the shift block connector approaches the base.

6. The quick insertion-and-removal device for the surgical instrument according to claim 5, wherein a plurality of protrusions are provided at intervals along a direction in which the shift block connector slides relative to the base.

7. The quick insertion-and-removal device for the surgical instrument according to claim 6, wherein the accommodating holes correspond to the protrusions in number and location.

8. The quick insertion-and-removal device for the surgical instrument according to claim 5, wherein a slideway extending along the motion direction of the joint unlocking member is opened in the base, and sides of the slideway are capable of guiding the sliding slidable portion.

9. The quick insertion-and-removal device for the surgical instrument according to claim 8, wherein an inclined surface is further provided on a bottom surface of the slideway, and the shift block connector is slidable along the inclined surface to approach a side of the base.

10. The quick insertion-and-removal device for the surgical instrument according to claim 3, wherein the outer sheath limiting assembly further comprises a detection element which is arranged within a sliding path of the limiting element and used to detect a stop position of the limiting element in the slide hole.

11. The quick insertion-and-removal device for the surgical instrument according to claim 2, wherein the outer sheath limiting assembly further comprises a first spring which is provided so that the limiting element can be retained in position of locking the outer sheath.

12. The quick insertion-and-removal device for the surgical instrument according to claim 11, wherein the outer sheath limiting assembly further comprises a blocking piece, and the first spring is compressed between the limiting element and the blocking piece.

13. The quick insertion-and-removal device for the surgical instrument according to claim 1, wherein the limiting element is provided with a first linked engagement portion, the joint unlocking member is provided with a second linked engagement portion, and the joint unlocking member and the limiting element are engageble with each other through cooperation between the first linked engagement portion and the second linked engagement portion.

14. The quick insertion-and-removal device for the surgical instrument according to claim 13, wherein one of the first linked engagement portion and the second linked engagement portion is configured as an insertion hole, and the other is configured as an insertion portion receivable within the insertion hole.

15. The quick insertion-and-removal device for the surgical instrument according to claim 1, wherein the joint unlocking member further comprises a sleeving portion for surrounding or being inserted in the sliding sleeve, and the sliding sleeve comprises a blocking portion engageble with the sleeving portion so that the sliding sleeve is slidable along with the joint unlocking member.

16. The quick insertion-and-removal device for the surgical instrument according to claim 1, wherein the push rod limiting assembly further comprises a second spring and a spring force from the second spring makes the sliding sleeve keep a tendency of locking the push rod.

17. The quick insertion-and-removal device for the surgical instrument according to any one of claims 1 to 16, wherein the push rod limiting assembly further comprises a push rod locking sleeve arranged in the sliding sleeve and a push rod locking member, and wherein the push rod is provided in the push rod locking sleeve;
the push rod locking member is capable of being constrained by the sliding sleeve between the push rod locking sleeve and the push rod, so that the push rod is locked relative to the push rod locking sleeve; and
when the joint unlocking member moves to the position of being engaged with the limiting element, the sliding sleeve releases the push rod locking member to unlock the push rod.

18. The quick insertion-and-removal device for the surgical instrument according to claim 17, wherein along a motion direction of the joint unlocking member, the push rod locking members are arranged in pairs and symmetrically distributed on two sides of the push rod.

19. The quick insertion-and-removal device for the surgical instrument according to claim 17, wherein the push rod locking member is configured as a spherical member, and the sliding sleeve is capable of pressing the spherical member into a locking groove of the push rod to lock the push rod.

20. The quick insertion-and-removal device for the surgical instrument according to claim 19, wherein a locking hole for accommodating the spherical member is defined in the push rod locking sleeve, and the spherical member is movably arranged within the locking hole.

21. The quick insertion-and-removal device for the surgical instrument according to claim 17, wherein along a sliding direction of the sliding sleeve relative to the push rod locking sleeve, the sliding sleeve has a locking section and an unlocking section, and wherein when the sliding sleeve slides to a position where the locking section corresponds to the push rod locking sleeve, the push rod locking member locks the push rod; and when the sliding sleeve slides to a position where the unlocking section corresponds to the push rod locking sleeve, the push rod locking member is released to unlock the push rod.

22. The quick insertion-and-removal device for the surgical instrument according to claim 17, wherein one of the push rod locking sleeve and the sliding sleeve is provided with a guiding rib, and the other is provided with a guiding groove in a sliding fit with the guiding rib.

23. The quick insertion-and-removal device for the surgical instrument according to claim 17, wherein two push rods are provided, each of which corresponds to one said push rod locking sleeve.

24. An executing mechanism of a surgical robot, comprising the quick insertion-and-removal device according to any one of claims 1 to 23 and the surgical instrument which comprises an outer sheath and two push rods slidably inserted in the outer sheath, with each of the push rods being connected to a swing member.

25. The executing mechanism of the surgical robot according to claim 24, wherein the base has a slide hole extending perpendicular to a sliding direction of the outer sheath; the outer sheath limiting assembly comprises a limiting element which is slidably connected to the slide hole, and the limiting element is provided with an outer sheath limiting hole; and an outer wall of the outer sheath is opened with an annular limiting groove for limiting part of a hole wall of the outer sheath limiting hole.

26. The executing mechanism of the surgical robot according to claim 24, wherein the executing mechanism further comprises two groups of push rod driving assemblies each corresponding to each of the push rods, the push rod driving assembly comprising:
a linear driving device arranged on the base; and
a loading seat connected to an output end of motion of the linear driving device for driving the push rod to axially slide.

27. The executing mechanism of the surgical robot according to claim 26, wherein the linear driving device is configured as a linear motor; and
the push rod driving assembly further comprises a guiding member which is arranged on the base and used to limit rotation of the loading seat along with the linear motor.

28. The executing mechanism of the surgical robot according to claim 26, wherein the push rod limiting assembly comprises two push rod locking sleeves arranged within the sliding sleeve, and in a locked state, the push rod can move with the push rod locking sleeve which is connected to the loading seat.

29. The executing mechanism of the surgical robot according to claim 28, wherein the push rod driving assembly further comprises an assembly transition plate, and the loading seat is connectable with the push rod locking sleeve through the assembly transition plate.

30. The executing mechanism of the surgical robot according to claim 26, wherein the executing mechanism further comprises two groups of push rod driving assemblies each corresponding to each of the push rods, the push rod driving assembly comprising:
a linear driving device directly arranged on the base; and
a push rod locking sleeve connected to an output end of motion of the linear driving device for driving the push rod to axially slide.

31. A surgical robot, comprising the executing mechanism according to any one of claims 24 to 30 and a telecentric manipulating mechanism, the telecentric manipulating mechanism comprising a movable platform, a static platform, and a plurality of telescopic units; two ends of each telescopic unit are respectively rotatably connected to the movable platform and the static platform, and the plurality of telescopic units are configured to move forward and backward in a cooperation manner to drive the movable platform to move relative to the static platform; and the executing mechanism is arranged on the movable platform, and the surgical instrument has a preset telecentric fixed point, and deflection of the movable platform is capable of driving the surgical instrument to swing around the telecentric fixed point.
